# EUROPEAN PATENT SPECIFICATION

(11) **EP 0 713 385 B1**
(45) Date of publication and mention of the grant of the patent: **24.03.1999**
(21) Application number: 94924868.6
(22) Date of filing: 10.08.1994
(51) Int. Cl.: A61K 7/50, A61K 7/48

(54) **CLEANSING COMPOSITIONS CONTAINING AN ALKANOATE AS CONDITIONER**
REINIGUNGSMITTEL DIE EINEM ALKANOAT ALS CONDITIONER ENTHALTEN
COMPOSITIONS NETTOYANTES RENFERMANT UN ALCANOATE A TITRE D'AGENT REVITALISANT

(30) Priority: 13.08.1993 GB 9316847; 13.08.1993 GB 9316848; 25.08.1993 GB 9317684
(43) Date of publication of application: 29.05.1996
(73) Proprietor: UNILEVER PLC, London EC4P 4BQ (GB); UNILEVER N.V., 3013 AL Rotterdam (NL)
(72) Inventor: BOWSER, Paul Anthony, Dorset House Latchford Road, Merseyside L60 3RW (GB); EDWARDS, Christopher John C., 2 The Avenue, West Yorkshire LS22 5BU BU (GB); GRIEVESON,Ailsa Pauline Hilary 60 St Andrews Road, Merseyside L63 3DJ (GB); HAGAN, Desmond Bernard, 35 Hookstone Drive, Merseyside L64 4TE (GB); LYLE, Ian, Gardner, Deeside Clwyd CH5 1XQ (GB); ROSSER, David, Arthur, Heswall Wirral Merseyside L60 4RD (GB); SCOTT, Ian, Richard, Allendale, NJ 07401 (US)
(74) Representative: Elliott, Peter William
(86) International application number: EP9402671
(87) International publication number: WO9505160

(56) References cited:
- EP-A- 0 007 785
- EP-A- 0 150 914
- EP-A- 0 261 812
- US-A- 4 105 783
- US-A- 4 198 311

## Description

This invention relates to cleansing compositions, especially foaming compositions which may be used for the cleansing of the skin and/or the hair.

The invention also relates to a method for the delivery of a hydroxyalkanoate for moisturising the skin and use of compositions to achieve this benefit.

### BACKGROUND TO THE INVENTION

Human skin consists essentially of two layers: the inner dermis and the outer epidermis, the former functioning mainly as a mechanical support for the latter.

The epidermis, which can be as little as 0.06 mm thick in the case of the eye lid to as much as 0.8 mm on the foot, itself comprises four or five layers, namely:
(i) the Stratum Malpigii, which is the germinative layer of cells at the base of the epidermis that adjoins the Dermis.
(ii) the Stratum Spinosum, the prickle cell layer which represents the first morphologically distinct stage in the differentiation of epidermal cells. It consists of numerous evenly spaced intercellular bridges - tonofilaments - each with a central thickening. The margins of several of these thickenings accounts for the appearance of desmosomes. The tonofilaments form the earliest precursor of keratin.
(iii) the Stratum Granulosum, the granular layer immediately above the prickle cell layer, which contains basophilic granules of keratohyalin. Also present in the Stratum Granulosum are the bridges (desmosomes and tono filaments seen in the prickle cell layers), but their close apposition renders them less visible.
(iv) the Stratum Lucidum, seen especially in the epidermis of the hand and foot, comprises cells which are of even thickness and essentially non-nuclear.
(v) the Stratum Corneum, which lies above the Stratum Lucidum (when present), forms the outermost layer of the epidermis. The Stratum Corneum is composed of dead, flat, fully keratinized cells which lie on top of one another to a depth of from 0.02 to 0.8 mm. The Stratum corneum also possesses lipid materials which effectively form a waterproof barrier to the external surface of the skin.

Beneath the epidermis is the dermis which is composed of collagen, usually accompanied by elastin and reticulin. These materials are fibrous proteins embedded in a mucopolysaccharide ground substance. Several cellular types, together with nervous and vascular networks, are found in the dermis, together with specialised appendages, including sweat glands, hair follicles with associated sebaceous glands.

A soft, supple and flexible skin has a marked cosmetic appeal and these characteristics are attributes of normal functioning epidermis, particularly with respect to the young human subject. The outer layer of the epidermis, i.e. the stratum corneum, can however become dry and flaky following exposure to adverse climatic conditions, or by excessive contact with detergents or solvents which results in a loss of skin moisture. Consequently, the skin can lose its soft, supple and flexible characteristics.

Emollients such as fats, phospholipids and stearols, have in the past been used to soften dry skin, but this can leave the skin greasy and unattractive. As an alternative, the topical application to the skin of classical humectants does not alleviate this problem, as these compounds are not particularly skin substantive and are generally rinsed from the skin during washing.

It is therefore apparent that there exists a continuing need for effective methods for treating dry flaky skin to restore its original soft, supple and flexible characteristics, and indeed for maintaining these attributes of normal functioning epidermis.

In an article by Baiocchi et al in Cosmetics and Perfumery 90, 31-34 (1975), it is stated that sodium stearoyl lactylate, when incorporated in a hand cream or lotion, results in a subjectively smooth and supple but not excessively greasy feeling when such creams or lotions are topically applied to the hands. However, the primary reason for including this lactylate in such formulations is to function as a very efficient emulsifier.

In an article by Osipow et al in Drug & Cosmet Ind, May 1969, 64ff, it is disclosed that sodium stearoyl lactylate may be used in oil-in-water cosmetic creams as the emulsifier to impart body, lubricity and opalescence to the cream. It is alleged that its absorption to the skin may enhance its softening action.

In another article by Murphy in Cosmetics and Toiletries 94, 43ff (1979), the sorption of acyl lactylates on the skin was examined by using pigskin as a model. It is described that sodium isostearoyl lactylate appears to reduce dryness and scaling of skin and restores a healthy texture to dry skin.

Murphy in Cosmetics and Toiletries 93, 31 (1978) discusses a systemable approach to skin moisturisation and concludes that a combination of the pyrrolidone carboxylic acid (PCA) sodium salt, sodium lactate and lactic acid can be used as effective humectants to hold moisture in the skin.

US 4,105,783 (Yu & Van Scott) discloses a therapeutic treatment of dry skin consisting of the topical application of a lotion, cream or ointment containing one or more α or β- hydroxy acids including glycolic acid and lactic acid.

EP 0 530 866 (Unilever) concerns novel sulfoxy alkanoates surfactants which it is believed are broken down by enzymes naturally present in the skin, or are naturally hydrolysed upon contact with the skin to yield "Benefit Reagents" e.g. Hydroxy acid and/or Fatty Alcohol.

EP 0 442 708 (Unilever) discloses cosmetic compositions containing 2-hydroxy alkanoic acids. Due to the presence of these acids in compositions, several benefits are imparted to the skin, such as an increase in the elasticity of the skin, particularly of the stratum corneum. Similarly, EP 0 007 785 (Unilever) discloses cosmetic compositions comprising 2-hydroxy alkanoic acids, which also give various skin benefits when topically applied to the skin.

However, the extent to which the moisturisation of skin, or its ability to remain moist without becoming dry, by topical application of so-called "moisturisers" as proposed by other workers in the field of cosmetic science, is not significant. A search has therefore been conducted for other active materials that can be employed in this way for enhancing the moisturisation of skin or, once moisturised, for restricting the extent to which skin moisture loss will occur.

It has long been recognised that cream or lotion formulations containing lactic acid, usually as lactate ions in products near neutral pH, when applied topically to the skin, can improve the flexibility and texture of the skin, and it is believed that lactate contributed to this effect. In studying this approach, we have applied such creams and lotions to the skin and have shown that although lactate can thereby be deposited on the surface of the stratum corneum which forms the outermost part of the skin, very little actually penetrates through the stratum corneum to the underlying regions of the epidermis, namely to the Stratum Granulosum and other strata below. This is thought to be due to the hydrophilic (i.e. lipophobic) property of lactate ions which renders them relatively incompatible with the lipids naturally present in the Stratum Corneum, and which thereby present a barrier to the adsorption of hydrophilic molecules.

While investigating the properties of derivatives of lactic acid that were more lipophilic than lactate itself, (e.g. sodium lactate) we discovered that a range of derivatives of lactic acid and its homologues are more readily adsorbed on contact with the skin and indeed migrate through the skin to reach the epidermis beneath the Stratum Corneum to an extent superior to lactate ions. We also made the discovery using labelled materials and radio tracer techniques, that these molecules were cleaved within the epidermis, most likely by the presence of endogenous esterases or other enzymes, to form hydroxyalkanoates ions deep in the epidermis, as far as the Stratum Malpigii.

We have further discovered that esters of hydroxyalkanoic acids, which are oily in nature, can be delivered to skin from cleansing compositions which include surfactants, so as to be of foaming character.

### DEFINITION OF THE INVENTION

Accordingly, this invention provides a foaming composition which comprises foaming surfactant together with a substituted alkanoate having the structure (1): 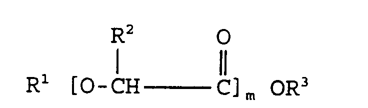 where
R¹ represents H- or 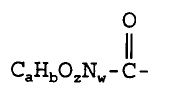
R² represents H-, or CₚH_{q}-
R³ represents CₓH_{y}O_{z}N_{w}-
a is an integer of from 1 to 20
b is an integer of from 3 to 41
p is an integer from 1 to 22
q is an integer of from 3 to 45
x is an integer of from 1 to 20
y is an integer of from 3 to 41
z is 0 or an integer of from 1 to 10
w is 0 or an integer of from 1 to 5
m is an integer of from 1 to 5
provided that when R¹ is H- and R² represents -H or -CH₃ then x is greater than 4.

Because the substitute alkanoate can penetrate into the skin, and there undergo enzymic cleavage, such a composition can function to deliver to the epidermis, as a moisturiser for the skin, the corresponding 2-hydroxyalkanoate having the structure (2): 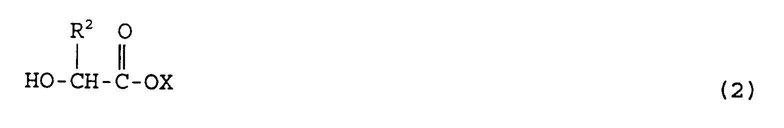 where
X represents -H or a counterion. The counterion may of course be any which is available in vivo.

The invention also provides a method for delivering to the skin a 2-hydroxy alkanoate having the structure (2): 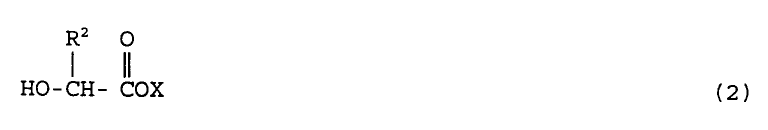 where
R² represents H- or -CₚH_{q}
X represents H- or a counterion
which comprises the steps of
i) applying topically to the skin a composition as specified above comprising the corresponding hydroxy alkanoate derivative having the structure (1),
ii) forming a lather on the hair or skin by massaging in the presence of added water, thereby to cleave the hair or skin, while allowing substituted alkanoate from the composition to penetrate through the stratum corneum at the scalp or other area of skin, and
iii) subsequently rinsing the lather from the hair or skin with water.

In the above formula (1) one possibility is that R¹ denotes an acyl group more especially an acyl group 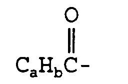 However, it is preferred that R¹ is -H.
a preferred group of compounds of the structure (1) are those where
R¹ represents H-
R² represents CₚH_{q}-
R³ represents CₓH_{y}-
p is from 1 to 12
q is from 3 to 25
x is from 1 to 18, and
y is from 3 to 37.

The invention is particularly concerned with compounds which incorporate alkyl groups of some length, so that they are oily in character. One group of such compounds has a group R² which is CₚH_{q}- in which p is at least 4.

Another group of such compounds has group R³ containing at least 6 carbon atoms, notably a group CₓH_{y}- in which x is at least 6.

### DETAILED DISCLOSURE

### The alkanoate derivatives

Substituted alkanoate derivatives for use in accordance with the invention are chosen from those having the structure (1) as herein defined.

When R² represents -CH₃, the compounds of structure (1) are derivatives of lactic acid. If R¹ is acyl, the compounds are frequently termed "lactylates". This name is used for compounds wherein m is one and also compounds wherein m has a higher value, that is to say both 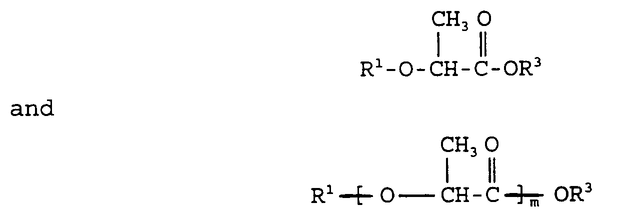 in which m is greater than one.

Examples of acyl derivatives of alkyl hydroxy alkanoates falling within the definition of structure (1) include:
n-octyl acetyl lactylate
lauryl acetyl lactylate
n-hexyl 2-acetoxybutanoate
lauryl 2-propionyloxybutanoate
n-butyl 2-acetoxyoctanoate
n-hexyl 2-(propionoyloxy)oactanoate
n-octyl 2-acetoxyoctanoate
lauryl 2-acetoxyoctanoate

Examples of alkyl hydroxyalkanoates that conform with structure (1) are those where:
R¹ in Structure (1) represents H-,
R² represents H- or -CH₃,
R³ represents CₓH_{y}-, and
m is 1.

Specific examples are:
n-hexyl glycate
n-octyl glycate
n-decyl glycate
n-dodecyl glycate
n-octadecyl glycate
n-hexyl lactate
n-octyl lactate
n-decyl lactate
n-dodecyl lactate
n-tetradecyl lactate
n-hexadecyl lactate
n-octadecyl lactate
2-octyldecyl lactate
octyl dodecyl lactate, and
palmitoyl glyceryl lactate.

Further examples of alkyl esters of hydroxyalkanoates, within structure (1) are:
those where
R¹ in structure (1) represents -H,
R² represents CₚH_{q}-
R³ represents CₓH_{y}-, and
m is 1 and p is at least 2.

Specific examples are:
methyl 2-hydroxybutanoate
n-butyl 2-hydroxybutanoate
n-hexyl 2-hydroxybutanoate
n-octyl 2-hydroxybutanoate
n-dodecyl 2-hydroxybutanoate
n-octadecyl 2-hydroxybutanoate
ethyl 2-hydroxyhexanoate
ethyl 2-hydroxyoctanoate, and
n-dodecyl 2-hydroxyoctanoate.

The amount of hydroxyalkanoate derivative to be employed in accordance with the invention is normally at least 0.5%, for instance 0.5 to 50%, preferably at least 1%, up to 20% or 30%, by weight of the composition.

A further constituent of the composition is surfactant. Generally this will be one or more surfactants from the classes of anionic and amphoteric. Possibly some non-ionic surfactant will also be included, although it is not preferred since it generally does not enhance foaming.

### Anionic surfactant

The surfactant may be soap, that is to say salt of carboxylic acid of 10 to 18 carbon atoms. If so, it may be found formed in situ by neutralisation of fatty acid during manufacture of a composition.

A non-soap anionic surfactant is preferably chosen from alkyl sulphate, alkyl ether sulphate, alkyl sulphonate, alkyl aryl sulphonate, olefin sulphonate, acyl sarcosinate, acyl tauride, acyl isethionate, nonoalkyl sulphosuccinate, dialkylsulphosuccinate, N-acylated α-amino acid, alkyl carboxylate, monoalkyl phosphate and dialkyl phosphate.

Specific examples of anionic surfactants include:
alkyl sulphates, such as sodium lauryl sulphate [eg. EMPICOL CX available from Albright & Wilson], and triethanolaminde lauryl sulphate [e.g. EMPICOL TL40/T, available from Albright & Wilson].
alkylether sulphates, such as sodium lauryl ether sulphate [eg. EMPICOL ESB70, available from Albright & Wilson].
alkyl sulphonates, such as sodium alkane (C₁₃₋₁₈) sulphonate [eg. HOSTAPUR SAS 30, available from Hoechst].
alkylaryl sulphonates, such as sodium alkyl benzene sulphonate [eg. TEEPOL CM44, available from Shell].
olefin sulphonates, such as sodium olefin sulphonate (C₅₋₁₈) [eg. HOSTAPUR OS, available from Hoechst].
acyl sarcosinates, having the structure: (51) 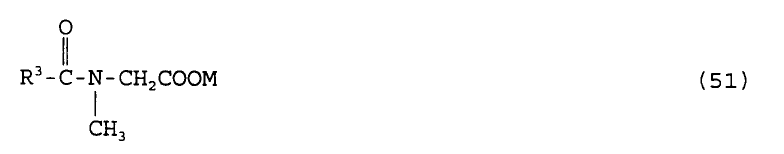 where
   R³ is chosen from C₆₋₁₄ alkyl, and
   M is a counterion chosen from alkali metals, ammonium and substituted ammonium such as alkanolammonium.

An example of an acyl sarcosinate having the structure (51), is sodium lauryl sarcosinate [eg. HAMPOSYL L-95, available from Grace].
acyl taurides, having the structure (52): 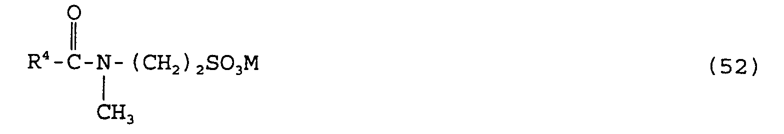 where R⁴ is chosen from C₈₋₁₈ alkyl

An example of an acyl tauride having the structure (52) is coconut methyl taurine [eg. FENOPEN TC 42, available from International Specialty Products].
acyl isethionates, having the structure (53): 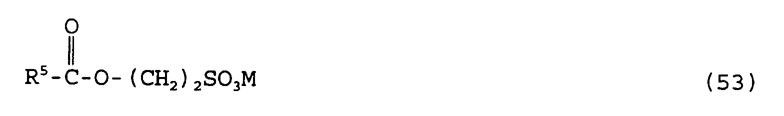 where R⁵ is chosen from C₈₋₁₈ alkyl.

An example of an acyl isethionate having the structure (53) is sodium acyl isethionate [eg. JORDAPON C1, available from Jordon].
monoalkyl sulphosuccinates, having the structure (54): 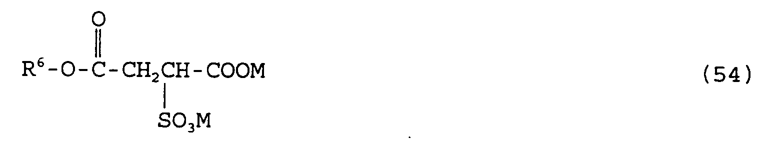 where R⁶ is chosen from C₁₀₋₂₀ alkyl.

Examples of monoalkyl sulphosuccinates having the structure (54) include:
sodium lauryl sulphosuccinate [eg. EMPICOL SLL, available from Albright & Wilson].
magnesium alkyl sulphosuccinate [eg. ELFANOL 616 Mg, available from AKZO].
sodium lauryl ethoxysulphosuccinate [eg. EMPICOL SDD, available from Albright & Wilson].
coconut monoethanolamide ethoxysulphosuccinate [eg. EMPICOL SGG].
disodium lauryl polyglycolether sulphosuccinate [eg. SURTAGENE S30, available from CHEM-Y].
polyethyleneglycol sulphosuccinate [eg. REWOPOL SBFA 30, available from REWO].
dialkyl sulphosuccinates, having the structure (55): 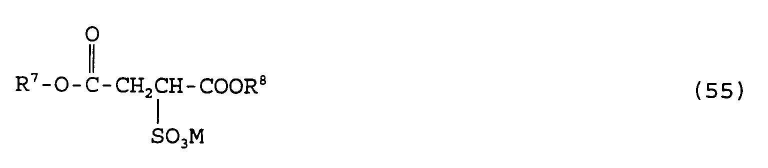 where R⁷ and R⁸ are the same or different, and are chosen from C₆₋₁₄ alkyl.

An example of a dialkyl sulphosuccinate having the structure (55) is sodium dilauryl sulphosuccinate [eg. EMCOL 4500, available from Witco].
N-acylated α-amino acids, such as sodium lauroyl glutamate [eg. ACYLGLUTAMATE LS-11, available from Ajinomoto Co. Inc].
alkyl ether carboxylates, such as C₁₂₋₁₄O(EO)₄OCH₂CO₂Na [eg. AKYPO RLM 38, available from Akzo].
monoalkyl phosphates and dialkyl phosphates, such as dioctyl phosphate.

### Amphoteric surfactant

The composition of the invention can also comprise an amphoteric surfactant. Suitable amphoteric surfactants are derivatives of aliphatic quaternary ammonium, phosphonium and sulphonium compounds, wherein the aliphatic radicals contain from 8 to 18 carbon atoms, and may be straight chain or branched, and further contain an anionic water-solubilising group, such as carboxyl, sulphonate, sulphate, phosphate or phosphonate.

Preferred amphoteric surfactants include:
Alkyl betaines, having the structure (58): 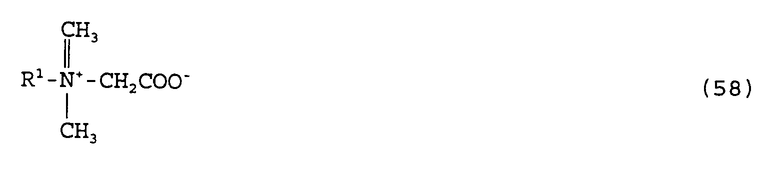 where R¹ is C₁₋₁₆ alkyl.

An example of an alkyl betaine having the structure (58) is lauryldimethyl betaine [eg. EMPIGEN BB, available from Albright & Wilson].
Alkylamidopropyl betaines, having the structure (59):

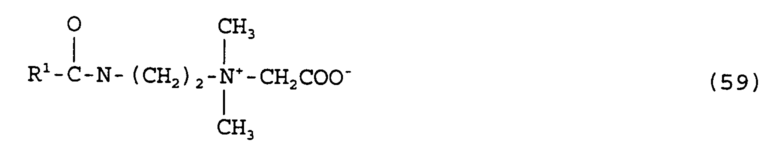

An example of an alkylamidopropyl betaine having the structure (59) is cocamidopropyl betaine [eg. TEGOBETAIN L7, available from Goldschmidt).
Alkylamphoglycinates or Alkylamphopropionates having the structure (60): 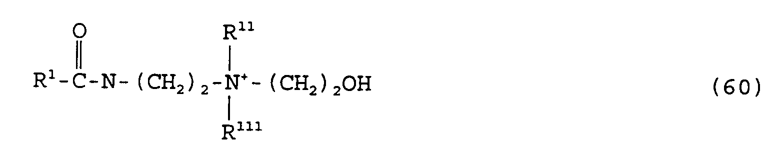 where R¹¹ is chosen from H, CH₂COO⁻ and (CH₂)₂COO⁻, and R¹¹¹ is chosen from CH₂COO⁻ and (CH₂)₂COO⁻

Suitable examples of compounds (60) are cocoamphoglycinate (available from International Specialty Products), and cocoamphopropionate.
Sultaines, having the structure (61): 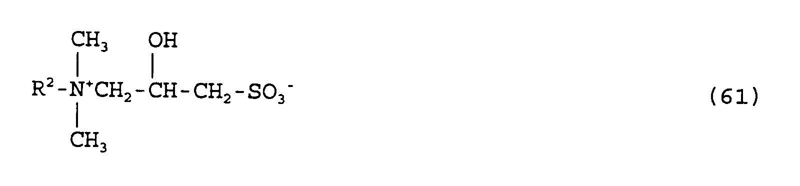 where R² is chosen from C₁₂₋₁₆ alkyl alkylamido groups.

An example of a sultaine having the structure (61) is cocamidopropylhydroxysultaine [eg. CYCLOTERIC BET-CS, available from Alcolac).

The most preferred amphoteric surfactant are lauryl dimethyl betaine and cocamidopropyl betaine.

Such amphoteric surfactants can contribute to the foaming of the skin cleansing composition, while ameliorating the harshness of the anionic surfactant.

### Nonionic surfactant

The composition of the invention can also comprise alkoxylated or glycosidic nonionic surfactant having an HLB of 8 or more. Above this value nonionics generally form clear isotropic solutions in combination with the other surfactants in the ranges defined above. Preferred nonionic surfactants are polyoxyethylene alkyl esters and polyoxyethylene alkyl ethers and alkyl polyglycosides.

A suitable example of a polyoxyethylene alkyl ester is that having the CTFA designation Polysorbate 80 which is a mixture of oleate esters of sorbitol and sorbitol anhydrides, condensed with approximately 20 moles of ethylene oxide. Also suitable is Polysorbate 20 which is a mixture of laurate esters or sorbitol and sorbitol anhydrides condensed with approximately 20 moles of ethylene oxide.

Polysorbate 80 and Polysorbate 20 are available commercially as TWEEN 80 and TWEEN 20 respectively, from ICI Americas.

Also suitable for use in the compositions of the invention is the polyethylene glycol ether of C₉₋₁₁ alcohol with an average of 8 ethoxy units, which is available commercially as NONIDET LE-8T or as SYNPERONIC 91-8T, and the polyethylene glycol ether of C₁₂₋₁₅ alcohol with an average of 9 ethoxy units which is available commercially as DOBANOL 25-9.

Particularly useful alkyl polyglycosides include the glycosides of glucose or glucose oligomers where the alkyl chain can be C₈₋₁₆ and the average number of glucose units is 1 to 2. A suitable example is ORAMIX NS 10 which is the glucoside of C₁₀₋₁₂ fatty alcohol with an average of about 1.5 glucose units.

A further possibility for use in compositions of the invention is high molecular weight silicone surfactant, such as a high molecular weight polymer of dimethyl polysiloxane with polyoxyethylene and/or polyoxypropylene side chains having a molecular weight from 10,000 to 50,000.

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the composition, more preferably 0.5 to 5%.

The amount of other surfactant present in a composition of this invention is normally at least 5% by weight. There will usually be at least 5% preferably at least 7% by weight of anionic and/or amphoteric surfactant, and not more than 40% in total of these ionic surfactants.

### Water or other vehicle

The composition of this invention will frequently include water or other liquid vehicle so that the composition is in the form of a liquid.

Particularly envisaged is to include water as a majority of the composition, for instance from 50 to 80% or 90% of the composition.

Other materials may be included in a composition of the invention and may form part of a vehicle for the alkanoate and surfactant. Materials which may be present include liquid or solid emollients, solvents, humectants, thickeners, and pearlescers.

Emollients are such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-o1, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, squalane, squalene, butyl myristate, isostearic acid, palmitic acid, isopropyl linoleate, decyl oleate and myristyl myristate.

Solvents are such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide and tetrahydrofuran.

The composition may be packaged in a pressurised container for an aerosol, spray or mousse, in which case, the composition will usually include one or more propellants, such as propane, butane, isobutane, dimethyl ether, carbon dioxide and nitrous oxide.

### Oily Materials

The composition according to the invention can optionally comprise one or more oils or other materials apart from alkanoates of structure (1) which have the properties of an oil and are immiscible with water, but can be emulsified in the surfactant-containing composition.

Examples of suitable oils include mineral oils and vegetable oils, silicone oils and oily materials such as those already proposed herein as emollients.

The quantity of oil, if present, is often at least 5% by weight, often remains a minority of the composition, in other words 5 to 50% by weight, preferably not more than 15%.

### Cationic polymer

A preferred constituent of compositions according to this invention is a cationic polymer, which can promote the deposition of the substituted alkanoate on the skin.

Preferred are cationic derivatives of guar gum. Most preferred are cationic guar gum derivatives given the CTFA designation guar hydroxypropyl trimonium chloride, available commercially for example as JAGUAR C13S, which has a low degree of cationic substitution and a high viscosity. Related suitable materials include that known as JAGUAR C15, having a moderate degree of substitution and a low viscosity, JAGUAR C17 (high degree of substitution), high viscosity) and JAGUAR C16 which is a hydroxypropylated cationic guar derivative containing a low level of substituent groups as well as cationic quaternary ammonium groups. Also suitable is JAGUAR 162 which is a high transparency, medium viscosity guar having a low degree of substitution.

The compositions of the invention contain from 0.01 to 3% by weight of cationic conditioning polymer, preferably from 0.05 or 0.1 to 2% by weight.

### Retinoids

The composition for use according to the invention optionally can also comprise a retinoid, such as retinoic acid or retinol (Vitamin A) and/or derivative thereof, further to enhance the benefits to skin.

In addition to retinol itself, examples of derivatives of retinol include:
Retinyl acetate
Retinyl butyrate
Retinyl propionate
Retinyl octanoate
Retinyl laurate
Retinyl palmitate
Retinyl oleate
Retinyl linoleate, and
Retinyl linolenate.

The amount of retinoid, when present in the composition according to the invention is from 0.01 to 10% and preferably 0.1 to 5% by weight of the composition.

### Tocopherol and Tocopheryl Esters

The composition for use according to the invention optionally can also comprise a tocopherol (vitamin E group), as an antioxidant for the composition, and to limit oxidative damage to skin. The vitamin E group comprises α-tocopherol, β-tocopherol, γ-tocopherol and δ-tocopherol. The composition according to the invention optionally can also comprise a tocopheryl ester, such as tocopheryl acetate.

The amount of a tocopherol, or ester thereof, when present in the composition according to the invention, is from 0.0001 to 20%, preferably from 0.0001 to 10% by weight of the composition.

Other materials which may be included in a composition according to this invention are humectants such as glycols, diglycerol, sorbitol, polyethylene glycol and 2-pyrrolidone-5-carboxylate.

### pH

The composition of the invention will normally have a pH value of from 4 to 9, preferably from 4.5 to 8.5. The pH can be adjusted as necessary by the addition of an alkali or acid as a pH adjustant, and/or by the addition of a buffer, such as a citrate buffer or a phosphate buffer.

### PRESERVATION OF THE COMPOSITION

The composition for use in accordance with the invention is preferably preserved against attack by bacteria, moulds and fungi and other microbial influences, in such a manner that it will enjoy an extended shelf life following manufacture and prior to sale and use. Ideally the composition will have an indefinite shelf life.

Examples of the methods that can be employed to achieve preservation of the composition, include the following:

### (i) Sterilisation

The composition according to the invention can be preserved by sterilization to remove or kill substantially all viable microbial contaminants. This can be achieved for example by irradiation using a lethal dose of gamma rays, by heat sterilization or by ultrafiltration using techniques that are well established in the pharmaceutical industry.

### (ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

### (iii) Water activity depressants

The composition according to the invention can also be preserved by the inclusion of a water activity depressant such as glycerol, propylene glycol, sorbitol, sugars and salts, for examples alkali metal halides, sulphates and carboxylates. When employing a water activity depressant, sufficient should be incorporated in the composition according to the invention to reduce the water activity (α_{w}) from 1 to < 0.9, preferably to < 0.85 and most preferably < 0.8, the lowest of these values being that at which yeasts, moulds and fungi will not proliferate.

### PROCESS

The invention also provides a process for preparing a composition according to the invention which comprises the steps of mixing an effective amount of an alkanoate as herein defined, together with surfactant and other cosmetically acceptable material if desired.

### Product Form and Container

The composition of the invention can be formulated as liquids, for example as a lotion, shampoo, milk or cream or put in a spray device such as an aerosol can containing propellant, or in a container fitted with a pump to disperse the liquid product. Alternatively, the compositions of the invention can be solid or semi-solid, for example sticks, creams or gels, for use in conjunction with a suitable applicator or simply a tube, bottle or lidded jar.

The invention accordingly also provides a closed container containing a composition as herein defined.

### USE OF THE COMPOSITION

The compositions of this invention when applied to the stratum corneum, will penetrate into the epidermis or cutaneous appendages, such as eccrine, apocrine and sebaceous glands, where hydrolysis by endogenous skin or microbial esterases will cleave the molecule to release in situ the corresponding hydroxy alkanoate, together with the fatty acid or fatty alcohol that forms the residue of the applied hydroxy alkanoate derivative.

The composition as applied to the skin surface will usually be a "rinse-off" product although a "wipe-off" product is possible.

When the composition for use in accordance with the invention is a "rinse-off" product, it will generally function as a skin cleanser. A suitable amount, for example 5 to 10 ml of the skin cleanser hydroxy alkanoate derivative, which can itself have surfactant properties, but is preferably accompanied by a co-surfactant, is applied to the skin and formed into a lather in the presence of water. After cleansing the skin, surplus product is generally rinsed from the skin and the skin is then dried. The rinse-off product can also be used for washing the hair or for cleansing the entire body surface, for example in the shower.

When the composition is a wipe-off product, it will also generally function as a skin cleanser, especially for removing make-up. A suitable amount, for example 0.5 to 5ml of the skin cleanser comprising the hydroxy alkanoate derivative can be applied to the skin, particularly where make-up is to be removed, and rubbed-in. The area of treated skin can then be wiped with a cloth, tissue or with cotton wool to remove surplus of the composition together with make-up that has been loosened from the skin.

One form of product according to this invention is a pre-shave product. Because men's shaving products are used regularly, they can serve to provide regular delivery of hydroxyalkanoate to the skin.

### EXAMPLES

### Example 1. Demonstrating Delivery of Lactate to the Epidermis

When an alkyl lactate is applied to the surface of the skin, it migrates by adsorption to a greater or lesser degree through the stratum corneum, dependent upon its lipophylicity. On contact with skin and/or microbial esterases, the molecule is cleaved and lactic acid/lactate is released within the stratum corneum, or cutaneous appendage and deeper in the epidermis.

The penetration of alkyl lactate and lactic acid in this manner can be determined by serial tape stripping and by biochemical assay of free lactate from skin cells adhering to the tape.

The tape employed in this test is Desquarne tape available from Diastron. Following topical application to skin of alkyl lactate, pieces of this tape are applied to the skin and then removed, and skin cells are assayed for lactate using Sigma 735-10 lactate assay kit. Thus, by repeated stripping of the same area of skin the degree of penetration of the topically applied alkyl lactate and its cleavage by epidermal esterases can be determined.

Lauryl lactate, having the formula 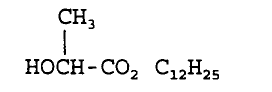 was dispersed in water at a concentration of 2% by weight.

This dispersion was tested on the volar forearm of a human volunteer. 100µl of the dispersion was applied to each of three areas (15cm²) on the volar forearm, rubbed in and allowed to dry. Ten Desquarne tape strips were taken from a fourth, untreated area ensuring that the tape strips were applied to the same site each time. After 1 hour, ten tape strips were taken from one treated area. Ten tape strips were taken from the other treated areas after 3 and 8 hours respectively. The strips taken after 1 and 3 hours were stored at room temperature until the end of the 8 hours, allowing ester cleavage to continue within the cells removed on the tape.

Each tape was placed in a labelled 1ml tube and 800µl of 5.00mM phosphate buffer, pH 7.00 was added to each tube. The tubes were then cycled from -20 to 20°C three times, with a minimum of 30 mins at each temperature, and then placed in an ultrasound bath for 30 mins. A bottle of Sigma 735-10 lactate reagent was then diluted with 5.00ml analar water, and 200µl aliquots were then transferred to each tube. The tubes were then allowed to stand at room temperature for a further 15 minutes. The buffer reagent mixture from each tube was then transferred to microcuvettes and the absorbtion of the solutions measured at 540nm using a spectrophotometer.

The results are set out in the following table. Tape 1 refers to the first tape strip taken from each site, and the values given are absorption units which are directly proportional to the concentration of L-lactic acid present on each tape.

| Tape No | No Treatment | 1 Hour | 3 Hours | 8 Hours |
|---|---|---|---|---|
| 1 | 0.172 | 0.289 | 0.282 | 0.260 |
| 2 | 0.209 | 0.361 | 0.250 | 0.210 |
| 3 | 0.197 | 0.217 | 0.252 | 0.250 |
| 4 | 0.199 | 0.214 | 0.265 | 0.222 |
| 5 | 0.189 | 0.192 | 0.485 | 0.259 |
| 6 | 0.177 | 0.201 | 0.487 | 0.230 |
| 7 | 0.183 | 0.179 | 0.271 | 0.241 |
| 8 | 0.177 | 0.177 | 0.246 | 0.234 |
| 9 | 0.157 | 0.211 | 0.254 | 0.194 |
| 10 | 0.177 | 0.169 | 0.269 | 0.449 |

It can be seen from the table that the application of lauryl lactate leads to penetration into the skin, and generation of lactate at the levels represented by all the tapes.

In an earlier investigation it had been shown that the application of sodium lactate itself leads to much less penetration into the skin, so that although it is able to increase the lactate content at a level near the surface, corresponding to the first two or three tapes only.

### Example 2

A composition, suitable for use as a facial wash, was prepared with the following formulation:

| | % w/w |
|---|---|
| Sodium lauryl ether sulphate | 16.0 |
| Cocoamidopropyl betaine | 2.2 |
| PEG Glycerol tallowate | 2.6 |
| Ethoxylated lauryl alcohol | 2.0 |
| Ethylene glycol monostearate | 1.0 |
| Lauryl lactate | 2.5 |
| Quaternised guar gum (Jaguar C13S) | 0.2 |
| Crosslinked polyacrylate thickener | 0.5 |
| Trisodium citrate | 0.37 |
| Potassium sorbate | 0.37 |
| Water | balance to 100% |

This composition had a pH of 5.5 and was an emulsion.

100µl of this emulsion was pipetted onto the surface of a piece of shaved pig skin, diluted with 500µl water and rubbed using a silicone rubber which was rotatated a controlled number of times with a controlled pressure against the pig skin. This mimics the generation of lather by massaging with the fingers, but standardises the conditions.

The skin was rinsed with 4 portions of water (500µl each) and dried. Five tape strips were taken from the centre of the skin, combined, and analysed for lactate content, both as salt and as deposited ester.

The same procedure was applied using similar compositions with higher concentrations of lauryl lactate.

The results were:

| | Absorption at 540nm |
|---|---|
| Water content | 0.18 |
| 2.5% lauryl lactate | 0.22 |
| 5.0% lauryl lactate | 0.28 |
| 10.0% lauryl lactate | 0.29 |

In a similar experiment, the concentration of lauryl lactate was maintained at 2% by weight, and the concentration of quaternised guar gum (Jaguar C13S) was varied.

The results were:

| | Absorption at 540nm |
|---|---|
| Water content | 0.18 |
| 0.2% Jaguar | 0.16 |
| 0.4% Jaguar | 0.19 |
| 0.6% Jaguar | 0.25 |

It is apparent from these results that lauryl lactate can be deposited on skin from a surfactant-containing composition. If there is a sufficient concentration of lauryl lactate, or if deposition is promoted by the presence of the cationic polymer, Jaguar C13S, the lactate content of the skin can be enhanced, relative to the lactate content when washing with water only.

### Example 3

Two compositions were prepared using a formulation similar to that in the preceding example, with the variation that one composition contained 2.0% lauryl lactate and 8% isopropyl palmitate whereas the other contained 10% isopropyl palmitate, without lauryl lactate, and served as a comparison with an equal content of oil.

A panel of volunteers washed their forearms with one or other of these two compositions twice a day for five days. Five tape strips were taken from each forearm at the beginning of the week, before the first wash, and at the end of the week, and assayed for lactate as in Example 1.

The comparative composition led to a small decrease in lactate (as salt) in the skin whereas the composition of the invention, including lauryl lactate, led to a small increase in skin lactate.

The invention is further illustrated by the following examples.

### Example 4. Mild Facial Cleanser

| Ingredients | % w/w |
|---|---|
| Lauryl lactate | 7.00 |
| Glycerol | 10.00 |
| Sodium cocoyl isethionate | 7.00 |
| Monoethanolamide sulphosuccinate | 3.00 |
| Cocamidopropyl betaine | 4.00 |
| Polyoxyethylene (20EO) 20 sorbitan monolaurate | 3.00 |
| Hydroxypropyl methylcellulose | 0.20 |
| Preservative | 0.20 |
| Perfume | 0.10 |
| Citric acid to pH 6.50 | |
| Water | to 100.00 |

### Example 5. Cleansing Mousse

| Ingredients | % w/w |
|---|---|
| Sodium lauryl ether sulphate (28%) | 18.00 |
| Sodium cocoamidopropyl betaine | 7.50 |
| Myristyl lactate | 5.00 |
| Glycerol | 10.00 |
| Ethanol | 5.00 |
| Vitamin E acetate | 0.10 |
| Cremophore RH410 | 0.50 |
| Redoderm LIS 80 | 1.00 |
| Preservative | 0.26 |
| Ammonium hydroxide (29%) to pH 7.00 | |
| Colourant | q.s |
| Perfume | q.s |
| Propane/Butane | 3.00 |
| Water | to 100.00 |

### Example 6. Clear Conditioning Shower Gel

| Ingredients | % w/w |
|---|---|
| Sodium laurylether sulphate | 13.0 |
| Cocoamidopropyl betaine | 2.00 |
| Glycerol | 5.00 |
| Hectorite clay | 1.00 |
| Hexyl 2-hydroxyoctanoate | 1.50 |
| Trisodium citrate | 0.37 |
| Potassium sorbate | 0.37 |
| Water | to 100 |

### Example 7. Facial Cream Wash

| Ingredients | % /w |
|---|---|
| Sodium cocoyl isethionate | 7.50 |
| Cocoamidopropyl betaine | 3.75 |
| Monoethanolamide sulphosuccinate | 3.75 |
| Glycerol | 8.00 |
| Stearic acid | 3.00 |
| Behenyl alcohol | 3.00 |
| Formaldehyde | 0.04 |
| Carpobol ETD 2020 | 0.50 |
| Isostearyl 2-hydroxyoctanoate | 5.00 |
| Polyethoxypropylene glycoldioleate | 0.50 |
| Sodium hydroxide | to pH 5.50 |
| Water | to 100 |

### Example 8. Body Conditioning Foam bath

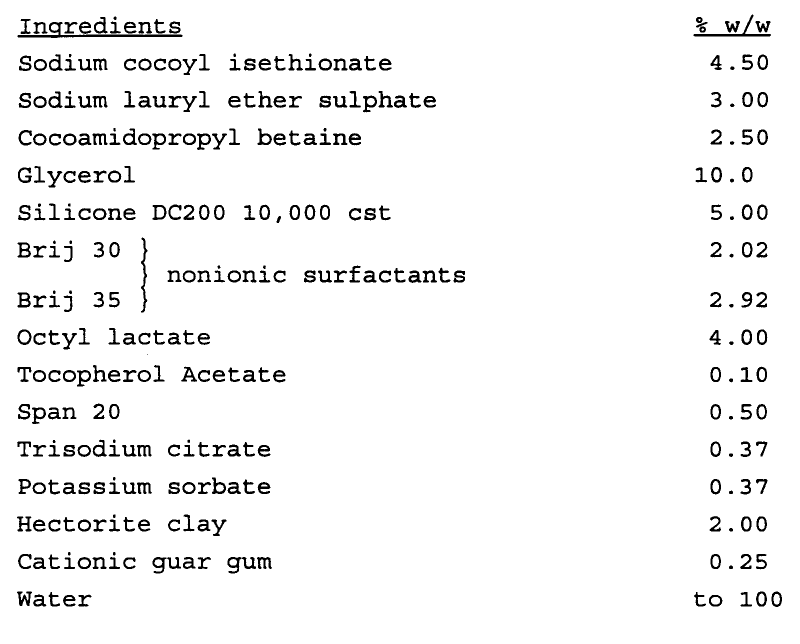

### Example 9. Liquid Soap

| Ingredients | % w/w |
|---|---|
| Sodium lauryl ether sulphate | 6.00 |
| Triethanolammonium N-lauroyl glutamate | 9.00 |
| Cocoamidopropyl betaine | 4.00 |
| Propyleneglycol 2-hydroxy isostearate | 1.00 |
| Butyl 2-hydroxyoctonate | 9.00 |
| Trisodium citrate | 7.00 |
| Preservative | 0.26 |
| Perfume | 0.15 |
| Triethanolamine to pH 7.00 | |
| Water | to 100.00 |

### Example 10. Smoothing Shaving Foam

| Ingredients | % w/w |
|---|---|
| Stearic acid | 3.27 |
| Palmitic acid | 3.51 |
| Lauric acid | 0.76 |
| Triethanolamine | 3.01 |
| Potassium hydroxide | 0.28 |
| Glycerol | 4.61 |
| Lauryl lactate | 1.30 |
| Tween 20 | 0.96 |
| Tocopherol acetate | 0.05 |
| Isostearyl lactylate | 0.10 |
| Silicone Fluid DC2-1865 | 1.50 |
| Silicone Fluid DC193 | 0.77 |
| CAP 48 (propellant) | 4.00 |
| Water | to 100 |

## Claims

1. A foaming cleansing composition which comprises foaming surfactant together with substituted alkanoate having the structure (1): 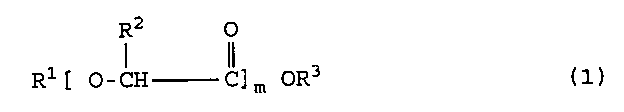 where
R¹ represents H- or 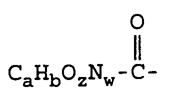
R² represents H- or CₚH_{q}-
R³ represents CₓH_{y}O_{z}N_{w}-
a is an integer of from 1 to 20
b is an integer of from 3 to 41
p is an integer of from 1 to 22
q is an integer of from 3 to 45
x is an integer of from 1 to 20
y is an integer of from 3 to 41
z is O or an integer of from 1 to 10
w is O or an integer of from 1 to 5
m is an integer of from 1 to 5
provided that when R¹ is H- and R² represents -H or -CH₃ then x is greater than 4.

2. a composition according to claim 1 where R¹ represents H- and m is 1.

3. A composition according to claim 1 or claim 2 where x is at least 6 unless R⁶ represents CₚH_{q}- in which p is at least 4.

4. A composition according to any one of claims 1 to 3 where
R¹ represents H-
R² represents CₚH_{q}-
R³ represents CₓH_{y}-
p is from 1 to 12
q is from 3 to 25
x is from 1 to 18, and
y is from 3 to 37.

5. A composition according to claim 4 in which the substituted alkanoate of the structure (1) is chosen from:
n-hexyl lactate
n-octyl lactate
n-decyl lactate
n-dodecyl lactate
n-tetradecyl lactate
n-hexadecyl lactate
iso-octadecyl lactate
n-octadecyl lactate
octyldodecyl lactate
methyl 2-hydroxybutanoate
n-butyl 2-hydroxybutanoate
n-hexyl 2-hydroxybutanoate
n-octyl 2-hydroxybutanoate
n-decyl 2-hydroxybutanoate
n-dodecyl 2-hydroxybutanoate
n-octadecyl 2-hydroxybutanoate
ethyl 2-hydroxyhexanoate
ethyl 2-hydroxyoctanoate
n-butyl 2-hydroxyoctanoate
n-hexyl 2-hydroxyoctanoate
n-octyl 2-hydroxyoctanoate
n-decyl 2-hydroxyoctanoate, and
n-dodecyl 2-hydroxyoctanoate.

6. A composition according to any one of the preceding claims in which the composition contains from 5 to 40% by weight of anionic and/or amphoteric surfactant.

7. A composition according to any one of the preceding claims in which the composition contains from 0.5 to 50% of the substituted alkanoate.

8. A composition according to any one of the preceding claims in which the composition contains a cationic polymer.

9. A composition according to any one of the preceding claims which contains water in an amount which is at least 50% by weight of the composition.

10. A method for delivering to the epidermis as a cosmetic treatment thereof a 2-hydroxyalkanoate having the structure (2): 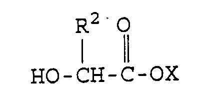 where
R² represents H- or CₚH_{q} as defined in claim 1
X represent H - or a counterion
which comprises the steps of
i) applying topically to the hair or skin a composition as defined in any one of the preceding claims,
ii) forming a lather on the hair or skin by massaging in the presence of added water, thereby to cleanse the hair or skin, while allowing substituted alkanoate from the composition to penetrate through the stratum corneum at the scalp or other area of skin, and
iii) subsequently rinsing the lather from the hair or skin with water.

11. Cosmetic use of a substituted alkanoate of structure (1) as defined in any one of claims 1 to 5 in a cosmetic compositon for delivering to the epidermis a 2-hydroxyalkanoate having the structure (2) as defined in claim 10.

12. Cosmetic use according to claim 11 wherein the said composition is in accordance with any one of claims 6 to 9.

## Patentansprüche

1. Eine schäumende Reinigungszusammensetzung, welche schäumendes Surfactant zusammen mit einem substituierten Alkanoat mit der Struktur (1): 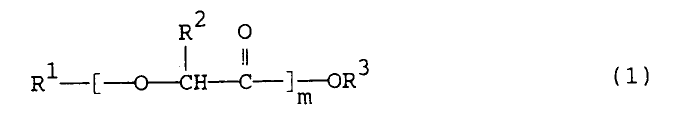 umfaßt,
worin
R¹ H- oder 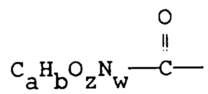 bedeutet,
R² H-, oder CₚH_{q}- bedeutet,
R³ CₓH_{y}O_{z}N_{w}- bedeutet,
a eine ganze Zahl mit einem Wert von 1 bis 20 ist,
b eine ganze Zahl mit einem Wert von 3 bis 41 ist,
p eine ganze Zahl mit einem Wert von 1 bis 22 ist,
q eine ganze Zahl mit einem Wert von 3 bis 45 ist,
x eine ganze Zahl mit einem Wert von 1 bis 20 ist,
y eine ganze Zahl mit einem Wert von 3 bis 41 ist,
z 0 ist oder eine ganze Zahl mit einem Wert von 1 bis 10,
w 0 ist oder eine ganze Zahl mit einem Wert von 1 bis 5,
m eine ganze Zahl mit einem Wert von 1 bis 5 ist,
vorausgesetzt, daß, wenn R¹ H- ist und R² -H oder -CH₃ bedeutet, dann x größer als 4 ist.

2. Eine Zusammensetzung nach Anspruch 1, worin R¹ H- bedeutet und m 1 ist.

3. Eine Zusammensetzung nach Anspruch 1 oder Anspruch 2, worin x zumindest 6 ist, es sei denn, daß R⁶ CₚH_{q}- bedeutet, worin p zumindest 4 ist.

4. Eine Zusammensetzung nach irgendeinem der Ansprüche 1 bis 3, worin
R¹ H- bedeutet,
R² CₚH_{q}- bedeutet,
R³ CₓH_{y}- bedeutet,
p von 1 bis 12 ist,
q von 3 bis 25 ist
x von 1 bis 18 ist und
y von 3 bis 37 ist.

5. Eine Zusammensetzung nach Anspruch 4, in welcher das substituierte Alkanoat der Struktur (1) ausgewählt ist aus:
n-Hexyllactat
n-Octyllactat
n-Decyllactat
n-Dodecyllactat
n-Tetradecyllactat
n-Hexadecyllactat
iso-Octadecyllactat
n-Octadecyllactat
Octyldodecyllactat
Methyl-2-hydroxybutanoat
n-Butyl-2-hydroxybutanoat
n-Hexyl-2-hydroxybutanoat
n-Octyl-2-hydroxybutanoat
n-Decyl-2-hydroxybutanoat
n-Dodecyl-2-hydroxybutanoat
n-Octadecyl-2-hydroxybutanoat
Ethyl-2-hydroxyhexanoat
Ethyl-2-hydroxyoctanoat
n-Butyl-2-hydroxyoctanoat
n-Hexyl-2-hydroxyoctanoat
n-Octyl-2-hydroxyoctanoat
n-Decyl-2-hydroxyoctanoat und
n-Dodecyl-2-Hydroxyoctanoat.

6. Eine Zusammensetzung nach irgendeinem der vorhergehenden Ansprüche, in welcher die Zusammensetzung von 5 bis 40 Gewichtsprozent von anionischem und/oder amphoterem Surfactant enthält.

7. Eine Zusammensetzung nach irgendeinem der vorhergehenden Ansprüche, in welcher die Zusammensetzung von 0,5 bis 50 % des substituierten Alkanoats enthält.

8. Eine Zusammensetzung nach irgendeinem der vorhergehenden Ansprüche, in welcher die Zusammensetzung ein kationisches Polymeres enthält.

9. Eine Zusammensetzung nach irgendeinem der vorhergehenden Ansprüche, welche Wasser in einer Menge enthält, die zumindest 50 Gewichtsprozent der Zusammensetzung beträgt.

10. Ein Verfahren zum Zuführen eines 2-Hydroxyalkanoats auf die Epidermis als eine kosmetische Behandlung derselben mit der Struktur (2): 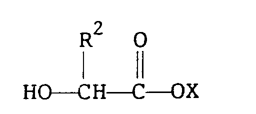 worin
R²H- oder CₚH_{q}, wie in Anspruch 1 definiert, bedeutet,
X H- oder ein Gegenion bedeutet,
welches die Stufen von
(I) örtlicher Anwendung einer Zusammensetzung auf das Haar oder die Haut, wie in irgendeinem der vorhergehenden Ansprüche,
(II) Bilden eines Schaums auf dem Haar oder der Haut durch Massieren in der Gegenwart von zugesetztem Wasser, um dadurch das Haar oder die Haut zu reinigen, während es dem substituierten Alkanoat von der Zusammensetzung ermöglicht wird, durch das Stratum corneum beim Skalp oder einem anderen Bereich der Haut einzudringen, und
(III) anschließendem Spülen des Schaums aus dem Haar oder der Haut mit Wasser
umfaßt.

11. Kosmetische Verwendung eines substituierten Alkanoats der Struktur (1), wie in irgendeinem der Ansprüche 1 bis 5 definiert, in einer kosmetischen Zusammensetzung zum Zuführen eines 2-Hydroxyalkanoats mit der Struktur (2) auf die Epidermis, wie in Anspruch 10 definiert.

12. Kosmetische Verwendung nach Anspruch 11, worin die Zusammensetzung in Übereinstimmung mit irgendeinem der Ansprüche 6 bis 9 ist.

## Revendications

1. Composition nettoyante moussante qui comprend un agent tensioactif moussant avec un alcanoate substitué ayant la structure (1) : 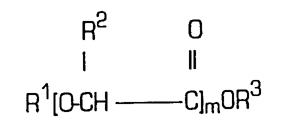 dans laquelle
R¹ représente H- ou 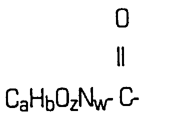
R² représente H- ou CₚH_{q}-
R³ représente CₓH_{y}O_{z}N_{w}-
a est un nombre entier compris entre 1 et 20
b est un nombre entier compris entre 3 et 41
p est un nombre entier compris entre 1 et 22
q est un nombre entier compris entre 3 et 45
x est un nombre entier compris entre 1 et 20
y est un nombre entier compris entre 3 et 41
z vaut 0 ou est un nombre entier compris entre 1 et 10
w vaut 0 ou est un nombre entier compris entre 1 et 5
m est un nombre entier compris entre 1 et 5
à condition que lorsque R¹ représente -H et R² représente -H ou -CH₃, alors x est supérieur à 4.

2. Composition selon la revendication 1, dans laquelle R¹ représente -H et m vaut 1.

3. Composition selon la revendication 1 ou la revendication 2, dans laquelle x vaut au moins 6 sauf si R⁶ représente CₚH_{q}- dans lequel p vaut au moins 4.

4. Composition selon l'une quelconque des revendication 1 à 3, dans laquelle
R¹ représente H-
R² représente CₚH_{q}-
R³ représente CₓH_{y}-
p est compris entre 1 et 12
q est compris entre 3 et 25
x est compris entre 1 et 18, et
y est compris entre 3 et 37

5. Composition selon la revendication 4, dans laquelle l'alcanoate substitué de structure (1) est choisi parmi :
le lactate de n-hexyle
le lactate de n-octyle
le lactate de n-décyle
le lactate de n-dodécyle
le lactate de n-tétradécyle
le lactate de n-hexadécyle
le lactate d'iso-octadécyle
le lactate de n-octadécyle
le lactate d'octyldécyle
le 2-hydroxybutanoate de méthyle
le 2-hydroxybutanoate de n-butyle
le 2-hydroxybutanoate de n-hexyle
le 2-hydroxybutanoate de n-octyle
le 2-hydroxybutanoate de n-décyle
le 2-hydroxybutanoate de n-dodécyle
le 2-hydroxybutanoate de n-octadécyle
le 2-hydroxyhexanoate d'éthyle
le 2-hydroxyoctanoate d'éthyle
le 2-hydroxyoctanoate de n-butyle
le 2-hydroxyoctanoate de n-hexyle
le 2-hydroxyoctanoate de n-octyle
le 2-hydroxyoctanoate de n-décyle, et
le 2-hydroxyoctanoate de n-dodécyle.

6. Composition selon l'une quelconque des revendications précédentes, dans laquelle la composition contient de 5 à 40 % en poids d'un agent tensioactif anionique et/ou amphotère.

7. Composition selon l'une quelconque des revendications précédentes, la composition contenant de 0,5 à 50 % en poids de l'alcanoate substitué.

8. Composition selon l'une quelconque des revendications précédentes, la composition contenant un polymère cationique.

9. Composition selon l'une quelconque des revendications précédentes qui contient de l'eau en une quantité qui est égale à au moins 50 % en poids de la composition.

10. Méthode de délivrance à l'épiderme à titre de traitement cosmétique pour celui-ci d'un 2-hydroxyalcanoate ayant la structure (2) 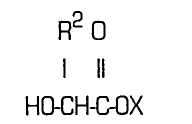 dans laquelle
R² représente H- ou CₚH_{q} tel que défini dans la revendication 1
X représente H- ou un contre-ion
qui comprend les étapes consistant
i) à appliquer par voie topique aux cheveux ou à la peau une composition telle que définie dans l'une quelconque des revendications précédentes,
ii) à former une mousse sur les cheveux ou la peau en massant en présence d'eau ajoutée, en nettoyant ainsi les cheveux ou la peau, tout en laissant l'alcanoate substitué de la composition pénétré à travers la couche cornée du cuir chevelu ou d'une autre zone de la peau, et
iii) à rincer ensuite la mousse des cheveux ou de la peau avec de l'eau.

11. Utilisation cosmétique d'un alcanoate substitué de structure (1) tel que défini dans l'une quelconque des revendications 1 à 5 dans une composition cosmétique délivrant à l'épiderme un 2-hydroxyalcanoate ayant la structure (2) telle définie dans la revendication 10.

12. Utilisation cosmétique selon la revendication 11, dans laquelle ladite composition est conforme à l'une quelconque des revendication 6 à 9.
